# EUROPEAN PATENT APPLICATION

(11) **EP 2 554 045 A1**
(43) Date of publication of application: **06.02.2013**
(21) Application number: 11176591.3
(22) Date of filing: 04.08.2011
(51) Int. Cl.: A01H 5/00, A01H 5/02, C12N 15/01, C12N 15/82

(54) **Method for systemically influencing processes in the male meiocyte**

(71) Applicant: Rijk Zwaan Zaadteelt en Zaadhandel B.V., 2678 KX De Lier (NL)
(72) Inventor: Kragler, Friedrich, 14467 Potsdam (DE); Kollwig, Gregor, 3040 Neulengbach (AT); Dirks, Robert Hélène Ghislain, 4731 GL Oudenbosch (NL)
(74) Representative: Van Someren, Petronella F. H. M.

(57) **Abstract**

The invention relates to a method for interfering with processes in the male meiocytes of a plant, by combining a first plant comprising a rootstock, which transgenically harbours a nucleic acid sequence, with a scion from a second plant grafted onto the rootstock of the said first plant, whereby the said transgenic nucleic acid sequence generates polynucleotide molecules in the rootstock of the first plant, which polynucleotide molecules are transported systemically across the graft junction to enter the male meiocytes produced by the scion of the second plant, and wherein the said systemically transported polynucleotide molecules are at least partially complementary to a transcript expressed in the male meiocytes produced by the scion of the second plant.

## Description

### FIELD OF THE INVENTION

This invention relates to a method for interfering with processes in the male meiocytes of a plant.

### BACKGROUND OF THE INVENTION

Sexual reproduction is arguably the most fundamental process in a flowering plant's life cycle. It provides a next generation of organisms, and through recombination (reshuffling) of the genetic features of the father and mother organism it allows the creation of genetic diversity. This diversity in the next generation allows plants to e.g. adapt better and more readily to changes in their biotic and abiotic environment.

Biological studies have led to a thorough understanding of the processes and factors that play important roles in sexual reproduction in angiosperms. Essential stages are:
- the formation and ripening of male gametes (microspores, pollen grains) and female gametes (egg cell inside an embryo sac inside an ovule) from diploid meiocytes. The formation of haploid gametes occurs via two meiotic divisions, during which the two parental genomes of the gamete-producing organism are recombined;
- pollination (the deposition of male gametes on the female reproductive organs/stigma), which can e.g. be mediated by wind, insects or other specialised pollinators, or simply by gravity, proximity or active pollination mechanisms when it concerns a self-fertilising species;
- the germination of pollen grains and the subsequent transfer of male nuclei (sperm cells) towards receptive and chemically attracting egg cells through a pollen tube;
- the release of sperm nuclei inside a receptive embryo sac, leading to double fertilisation, in which one sperm cell fuses with the egg cell to give rise to a diploid embryo, and the other sperm cell fuses with the diploid central cell, to give rise to triploid endosperm;
- mitotic divisions and developmental patterning of the zygote, leading to the formation of an embryo.

For the purpose of this invention the first stage is most important: the formation of gametes, in particular male gametes, from meiocytes. In angiosperms the male gametes or microspores are formed inside anthers. They originate from diploid pollen mother cells (a type of meiocyte) that undergo meiosis, which encompasses two rounds of meiotic division. The final result of meiosis is a set of four haploid microspores for each pollen mother cell, and these microspores each develop further by undergoing two mitotic divisions. The first of these divisions is asymmetric, and it gives rise to a vegetative cell and a generative cell, while the second mitosis concerns the division of the generative cell. The result is a pollen grain that contains two sperm cells, in a unique constellation, as the sperm cells are both located inside the cytoplasm of the vegetative cell.

The timing of the second mitotic division varies greatly in the plant kingdom, and the second pollen mitosis can either occur prior to anthesis (as is the case in e.g. *Arabidopsis* and rice), or after anthesis during pollen tube growth (e.g. in tomato and tobacco). Depending on the species, angiosperm flowers can thus shed either tricellular (containing one vegetative nucleus plus two generative nuclei) or bicellular pollen grains (containing one vegetative nucleus plus one generative nucleus).

Meiotic cell division is of fundamental importance during gamete formation, as it provides a means for reshuffling the parental genetic information so that new genetic combinations arise, and a means for "undoubling" the genome in haploid gametes. Meiocytes have received considerable research attention, because if one would be able to influence the molecular, physiological and/or developmental processes that take place inside a meiocyte, one could potentially have a huge impact on sexual reproduction, on the transmission of genetic material to the next generation, and even on evolution.

It is known that in plants RNA-based silencing signals spread systemically to distant organs. For long-distance transport of such RNA-based signals, flowering plants use a specialized vascular system named phloem. The phloem facilitates the directional transfer of small molecules such as ions, sugars, nucleotides, hormones, amino acids, and proteins from source (producing) tissues to sink (consuming) tissues. In a number of plant species small polynucleotide molecules (typically RNA molecules with a size of 18 to 27 nucleotides), derived from specific gene products, were shown to be present in the phloem sap and suggested to relocate following the source-sink flow.

It is known from the literature that a long-distance transport pathway allows plants to induce systemic post-transcriptional gene silencing (PTGS) associated with small interfering RNA (siRNA) molecules.

PTGS - associated with siRNA - is reset by an unknown mechanism and is re-established in the following generation. PTGS-resetting seems to take place during meiosis and can be established again in gametes, as shown in studies on transposon activity in male gametophytes.

siRNAs targeting transposable element transcripts are activated in the vegetative nucleus and transferred into fertilizing sperm cells, where they down-regulate transposon activity. Thus, within pollen grains and fertilizing pollen tubes an siRNA-mediated silencing machinery is active. The PTGS mechanism is also functional in sporophytic tissues, as anther-specific genes could be silenced. Studies conducted with transgenic GFP-expressing lines, in which siRNA-associated spread of silencing was induced by overexpression of transcripts containing GFP sequences, suggested systemic spread of siRNA signals into strong sink tissues such as newly forming leaves.

However, it seems that not all sink tissues can receive PTGS signals. Systemic siRNA-mediated GFP-silencing was reported to be very limited in growing flower buds, and silencing of GFP in the meiotic (sporogenic) tissues of anthers or carpels has never been observed.

In the prior art it is thus presumed that meiocytes are inaccessible for long-distance mobile signals, and that a barrier exists that protects the future microspores in their earliest stages of development.

In the research that led to the present invention it was however found how meiocytes can be targeted with systemic polynucleotide signals from a transgenic rootstock, without rendering transgenic the shoot grafted thereon (scion), or the meiocytes or microspores produced by the scion, or plants derived from these microspores.

The present inventors found how to interfere with (molecular) processes in the male meiocytes, through the said polynucleotide signals that are transported into the male meiocytes as a systemic signal.

In the prior art it is also known that the overexpression of RNAi constructs with constitutive promoters can lead to silencing of gene expression in the male meiocytes (Dirks R et al, 2009, Plant Biotechnol J 7: 837-845; Reverse Breeding: WO03/017753). However, a considerable drawback of this approach is the fact that the organism producing the male meiocytes, as well as at least 50% of the microspores produced after meiosis, are transgenic. For regulatory, efficiency and practical reasons it is very desirable to develop a method for producing transgene-free plants from spores that originated from meiocytes in which molecular recombination processes have been interfered with. Such microspores can e.g. be used for the production of transgene-free doubled haploid plants (DHs).

The prior art discloses various methods for the production of haploid plants from microspores, such as *in vitro* microspore culture (regeneration of haploid plantlets from microspores, and subsequent genome doubling with e.g. colchicine), and the method disclosed in WO2011044132, which involves pollination of a haploid-inducer line, which after fertilisation eliminates the maternal chromosomes from the zygote, leading to the formation of viable seeds containing a haploid embryo that is genetically identical to the microspore used for pollination and fertilisation.

In the research leading to this invention it was thus surprisingly found that siRNA signals, generated in a mature rootstock, can systemically penetrate and be active in apical sporogenic cells, in particular meiocytes. This was demonstrated by grafting a non-transgenic scion onto a transgenic rootstock harbouring an RNAi construct and producing siRNA molecules, and by observing silencing phenotypes of genes in the male meiocytes formed by the non-transgenic scion. Such systemic siRNA molecules targeting meiotic tissue can thus affect gene expression in the progeny.

The siRNA molecules, generated from the RNAi construct in the transgenic rootstock, can thus be transmitted through long-distance transport across a graft junction, and throughout the scion. The current research demonstrated that the polynucleotide (siRNA) molecules generated from these constructs can subsequently be forced to enter into the male meiocytes and effect gene silencing therein, or interfere in another way with transcripts or genomic material in these meiocytes. Until now it was deemed impossible to import molecules into early developing meiocytes or microspores through long-distance transport. Surprisingly, within a flower the siRNA molecules are preferentially targeted towards the anthers and the meiocytes therein, whereas the female meiocytes (megaspore mother cells contained in the ovary) do not receive the siRNA signal.

This invention can be applied to interfere with all molecular processes that take place in male meiocytes, such as meiosis, chromosome recombination, and homologous recombination, and also to interfere with the physiology of microspores, such as e.g. the responsiveness to androgenetic stimuli of microspores derived from these meiocytes. "Interference" can either refer to suppression or blocking of these processes, or to enhancement thereof. It also allows specific alteration of the genetic material that is transferred to the next generation, e.g. through site-directed mutagenesis (during the stage in which homologous recombination takes place spontaneously in meiocytes) in genes and/or regulatory (*cis*-acting) sequences.

Importantly, application of this invention does not render the male meiocytes, the microspores developing therefrom or the scion transgenic, as only the root stock harbours a transgene. The microspores and the scion only contain transiently expressed transgene-derived molecules, which are not passed on to the next generation. Only the molecular effects brought about by the transgene-derived molecules are transmitted to the next generation. The use of transgenes expressing dominant-negative factors that can systemically move across graft junctions into transgene-free plant material allows researchers to take full advantage of transgene technology to impose novel traits onto crop plants, without rendering them stably transgenic.

Specific mutations can be introduced into a plant genome through homologous recombination (Paszkowski J et al, 1988, EMBO J 7: 4021-4026; Mengiste T and Paszkowski J, 1999, Biol Chem 380: 749-758; Vergunst AC and Hooykaas PJJ, 1999, Crit Rev Plant Sci 18: 1-31) or through oligonucleotide-based mutation induction (Oh TJ and May GD, 2001, Curr Opin Biotechnol 12: 169-172; KeyBase technology: WO2010074562, EP2167660, WO2007073149).

The method of this invention can be used for plant breeding purposes. Current breeding methods are slow and take >5-10 years for cultivar development. One of the disadvantages is the inefficiency of trait selection in early generations. This problem can be overcome by inducing doubled-haploid production in non-transgenic scions by grafting them onto compatible transgenic root stocks, as illustrated by example 3 below.

The present invention can be applied to all plant species that can be grafted and transformed. Crop species on which this invention can be practised include e.g. tobacco, poplar, maize, wheat, barley, rice, sorghum, sugar beet, oilseed rape, ryegrass, sunflower, soybean, tomato, cucumber, gherkin, corn salad, spinach, pepper, petunia, potato, eggplant, melon, carrot, radish, lettuce, vegetable *Brassica* species (cabbage, cauliflower, broccoli, kohlrabi, Brussels sprouts), leek, bean, pea, endive, chicory, onion, strawberry, fennel, table beet, celery, celeriac, asparagus, sunflower, and grape vine.

The present invention thus relates to a method for interfering with processes in the male meiocytes of a plant, by combining a first plant comprising a rootstock, which transgenically harbours a nucleic acid sequence, with a scion from a second plant grafted onto the rootstock of the said first plant, whereby the said transgenic nucleic acid sequence generates polynucleotide molecules in the rootstock of the first plant, which polynucleotide molecules are transported systemically across the graft junction to enter the male meiocytes produced by the scion of the second plant, and wherein the said polynucleotide molecules are at least partially complementary to a transcript expressed in the male meiocytes produced by the scion of the second plant.

Expression of the transgenic nucleic acid sequence in the first plant is achieved by operably linking the said nucleic acid sequence to a promoter sequence that confers a ubiquitous or systemic expression profile, or a tissue- or cell-type-specific expression profile onto the said transgenic nucleic acid sequence. For this purpose the transgenic nucleic acid sequence is cloned into a transgenic construct, downstream (at the 3' end) of a promoter sequence that has the ability to drive the expression of the said nucleic acid sequence in plants. Promoter sequences differ in their expression patterns in plant tissues, and some have the ability to drive gene expression in all tissues (ubiquitous expression), while others have a (spatio-temporally) more narrow expression pattern, or a very specific expression pattern that is restricted to a specific tissue type or cell type.

Examples of promoter sequences that confer a ubiquitous expression profile onto a transgenic nucleic acid sequence are e.g. the cauliflower mosaic virus 35S promoter (CaMV 35S), selected ubiquitin promoters, selected actin promoters, and many others. Examples of tissue- or cell-type-specific promoters are also known to the skilled person.

For the purpose of this invention, the promoter sequence confers onto the said transgenic nucleic acid sequence an expression profile that encompasses the rootstock. This is desired because only the rootstock is used from the said first plant harbouring the transgenic nucleic acid sequence, and therefore the said nucleic acid sequence should be expressed at least in the rootstock of the said first plant. The rootstock encompasses the source tissues from which phloem sap is transported to the sink tissues, and more specifically to the sink tissues of the scion. A rootstock comprises the roots and may in addition comprise a part of the stem and some (mature) leaves. According to the invention, the expression product of the transgenic nucleic acid sequence can be produced in the complete rootstock or in a part thereof, for example by using a root-, leaf- or stem-specific promoter.

In one embodiment the expression of the transgenic nucleic acid sequence in the rootstock of the first plant is constitutive. In another embodiment, the expression of the transgenic nucleic acid sequence in the rootstock of the first plant is transient. Transient expression can e.g. be achieved by use of an inducible promoter, selected from the group comprising heat-inducible promoters, chemical-inducible promoters, steroid-inducible promoters and alcohol-inducible promoters. Transient expression can be achieved by using an inducible promoter or by transiently introducing the transgenic construct (with a strong promoter) into the root stock instead of stably integrating the transgene in the rootstock genome. Such transient transformation can for example be achieved by *Agrobacterium* infiltration via the stomata of the leaves that remain attached to the rootstock after grafting of the scion onto the rootstock.

In one embodiment, interfering with processes in the male meiocytes consists of the suppression or blocking of processes in the male meiocytes. In another embodiment, interfering with processes in the male meiocytes consists of the enhancement of processes in the male meiocytes. When interfering consists of the suppression or blocking of processes in the male meiocytes, this can be achieved by destabilizing the target gene mRNA by means of polynucleotide molecules that are at least partially complementary to the target gene mRNA or transcript and that have been generated from the transgenic nucleic acid sequence. The polynucleotide molecules can be selected from the group comprising miRNA, antisense RNA, RNAi molecules, siRNA molecules, Virus-Induced Gene Silencing (VIGS) molecules, co-suppressor molecules or RNA oligonucleotides.

In one embodiment the said transgenic nucleic acid sequence is stably integrated into the genome of the first plant. In another embodiment the said transgenic nucleic acid sequence is transiently present in the rootstock of the first plant.

In one embodiment the said polynucleotide molecules are generated and expressed constitutively in the first plant, preferably in an expression domain that comprises the roots. This implies that no external treatment is required for the generation and expression of the polynucleotide molecules. The properties of the promoter sequence to which the transgenic nucleic acid sequence is operably linked determine the temporal and spatial pattern of the generation and expression of the polynucleotide molecules.

In another embodiment the said polynucleotide molecules are generated and expressed transiently in the first plant, preferably in an expression domain that comprises the roots. This implies that external factors or treatments can be used to induce and/or modulate the temporal and/or spatial pattern of the generation and expression of the polynucleotide molecules, by virtue of the effect these external factors or treatments have on the activity of the promoter sequence to which the transgenic nucleic acid sequence is operably linked.

In this embodiment the transient expression of the polynucleotide molecules in the first plant is achieved by use of an inducible promoter, selected from the group comprising heat-inducible promoters, chemical-inducible promoters, steroid-inducible promoters and alcohol-inducible promoters. Treatment of the rootstock with the agent(s) and/or condition(s) that induce the activity of the inducible promoter (such as a heat shock or heat treatment, specific chemicals, specific steroids such as dexamethasone, or alcohol) activates the inducible promoter, which results in the transient expression of the polynucleotide molecules in the first plant, preferably in an expression domain that comprises the roots.

The polynucleotide molecules derived from the transgenic nucleic acid sequence are at least partially complementary to a transcript expressed in the male meiocytes produced by the scion of the second plant. In one embodiment, the polynucleotide molecules are at least partially complementary to the transcript (transcribed nucleic acid sequence or mRNA) of a gene that is involved in chromosome recombination. In this embodiment, the gene is suitably selected from the group comprising *SPO11, MER1, MER2, MRE2, ME14, REC102, REC104, REC114, MEK1*/*MRE4, RED1, HOP1, RAD50, MRE11, XRS2, PRD1, PRD2, PRD3, PHS1, NBS1, COM1, RHD54*/*TID1, DMC1, SAE3, RED1, HOP1, HOP2, MND1, REC8, MEI5, RAD51, RAD52, RAD54, RAD55, RAD57, RPA1, SMC3, SCC1, MSH2, MSH3, MSH6, PMS1, SOLODANCERS, HIM6, CHK2, SGS1, MSH4, MSH5, MER3*/*RCK, ZIP1, ZIP2, ZIP3, ZIP4, PTD, SHOC1, ZYP1, MLH1, MLH3,* or their functional homologues.

In another embodiment, the polynucleotide molecules that are generated by the transgenic nucleic acid sequence in the rootstock of the first plant and that are transported systemically across the graft junction to enter the male meiocytes produced by the scion of the second plant, are DNA polynucleotides. These DNA polynucleotides typically have the same size as the RNA polynucleotides of the previous embodiments, i.e. between about 18 nucleotides and about 27 nucleotides.

This invention further relates to a method for specifically altering the genome sequence of male meiocytes of a plant, by combining a first plant comprising a rootstock, which transgenically harbours a nucleic acid sequence, with a scion from a second plant grafted onto the rootstock of the said first plant, whereby the said transgenic nucleic acid sequence generates polynucleotide molecules in the rootstock of the first plant, which polynucleotide molecules are transported systemically across the graft junction to enter the male meiocytes produced by the scion of the second plant, and wherein the said polynucleotide molecules are at least partially homologous to a DNA fragment contained in the genome of the second plant.

In this embodiment advantage is taken of homologous recombination in the male meiocyte, to effect targeted nucleotide exchange in the genome sequence of the male meiocyte. This can e.g. be achieved through the presence of small DNA polynucleotides in the male meiocytes during the stage in which chromosome recombination occurs, and in which the chromosomes are more easily accessible. Specific mutations can be introduced into a plant genome through homologous recombination (Paszkowski J et al, 1988, EMBO J 7: 4021-4026; Mengiste T and Paszkowski J, 1999, Biol Chem 380: 749-758; Vergunst AC and Hooykaas PJJ, 1999, Crit Rev Plant Sci 18: 1-31) or through oligonucleotide-based mutation induction (Oh TJ and May GD, 2001, Curr Opin Biotechnol 12: 169-172; KeyBase technology: WO2010074562, EP2167660, WO2007073149). The polynucleotides used for this purpose are essentially homologous to a genomic sequence, except for one or more mismatches located essentially in the middle of the targeted sequence in the genome intended to create a targeted nucleotide-exchange or point-mutation in the genome.

The invention will be further illustrated in the Examples that follow and that are not intended to limit the invention in any way. In the Examples, reference is made to the following figures.

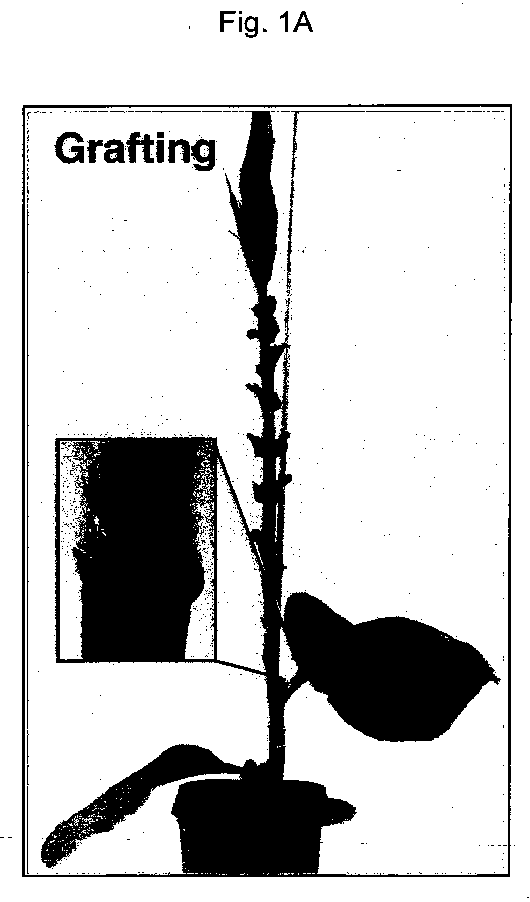

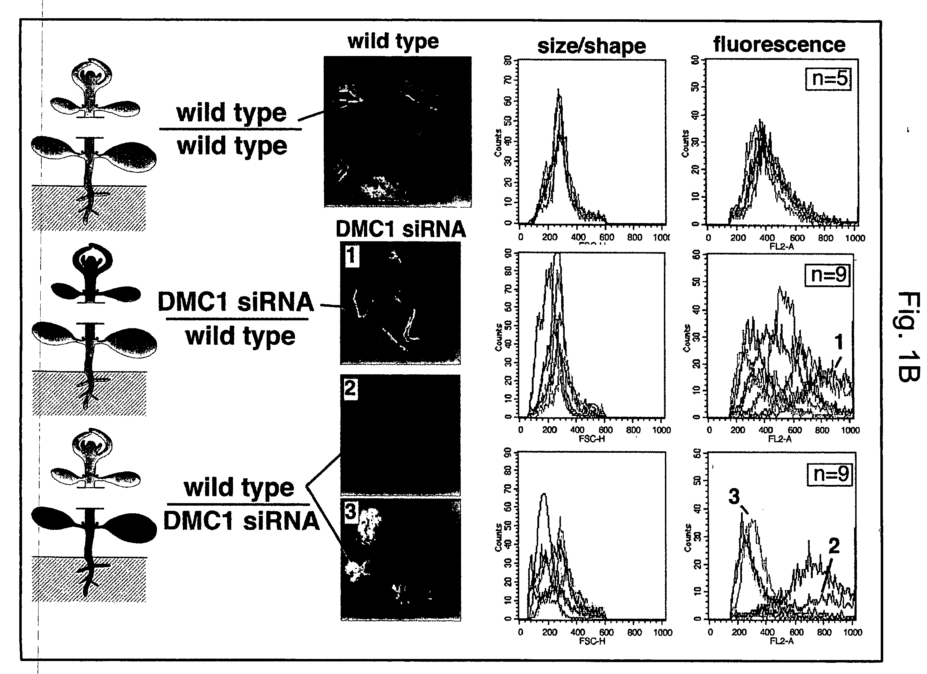

**Figure 1**: **(a)** Representative photograph of a grafted tobacco plant. A non-transgenic scion was grafted onto a transgenic rootstock, and all leaves were removed from the scion, to force the phloem-mobile silencing signal from source tissues (the rootstock) into the scion's apical region (sink tissues). **(b)** A schematic drawing of the graft combinations used in the cytometric (FACS) analysis of pollen harvested from grafted plants. The histograms indicate the size (scattered light) and fluorescence intensity of Propidium Iodide-stained pollen coat and nuclei. The numbers obtained by control grafts (wild type/wild type) are highly reproducible. In contrast, the size and DNA content of pollen from *DMC1* siRNA transgenic scions (reverse graft) or wild-type scions grafted on *DMC1* siRNA lines (true graft) is significantly altered. The numbers refer to representative images of scion anthers forming: male sterile flowers producing no or low quantities of aberrant pollen (numbers 1 and 2), and anthers with regular pollen production (number 3).

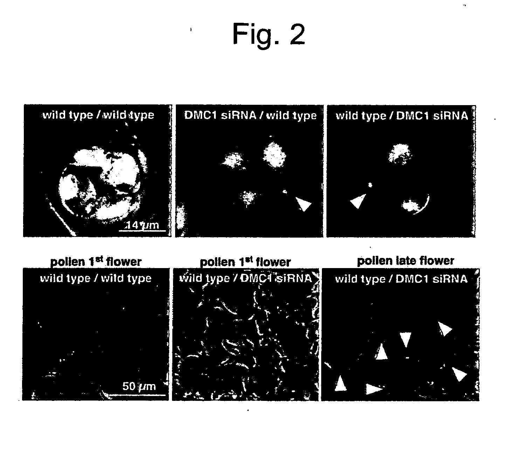

**Figure 2****: (Upper panel)** Microscopic images of DAPI stained nuclei (blue) in microspore tetrads isolated from scion flowers. Note the appearance of unbalanced tetrads (arrowhead) on *DMC1* siRNA-producing scions (middle image) and on wild-type plants grafted onto *DMC1* silencing lines (right image). **(Lower panel)** Scanning electron microscopy images of pollen harvested from scion flowers. Pollen grains from control grafts (wild type/wild type) were normal in shape and size. In contrast, wild-type scions grafted on transgenic *DMC1* silencing lines produced misshaped pollen grains that varied in size. Note that within one shoot the number of misshaped pollen was higher in primary flowers than in late flowers. This observation correlated with the sterile anther phenotype appearing on the first flower formed on wild-type scions grafted on *DMC1* siRNA lines.

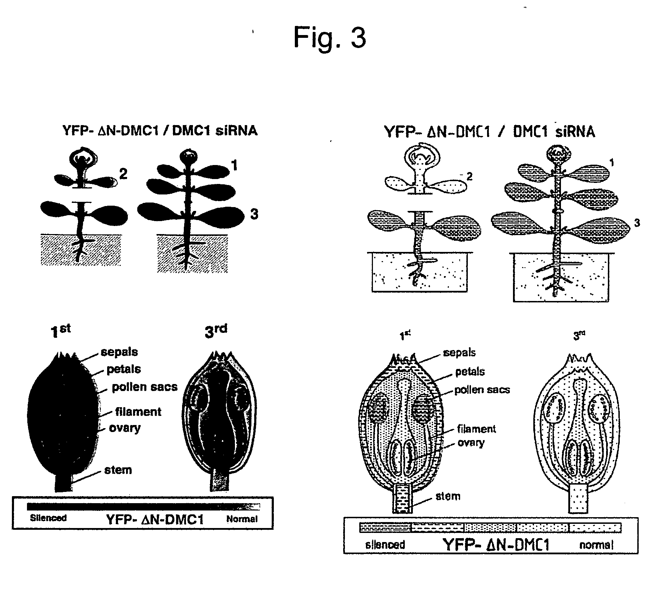

**Figure 3****:** Schematic drawing of the observed spread of the *DMC1* silencing signal in 35S::YFP-ΔN *DMC1* flower buds, both in color (left) and black-and-white (right).Green or dotted parts indicate parts of the plant where no silencing of the YFP reporter construct was observed. Blue or striped parts indicate the presence of *DMC1* silencing (=disappearance of YFP signals). *DMC1* silencing (blue/dotted) was observed in the epidermis of sepals and carpels, and in the sporogenic tissue of anthers. No silencing of the YFP reporter construct was observed in female organs such as pistils, carpels or ovules (green/striped), or in later forming flower buds on an inflorescence. Note that the observed spread of *DMC1* silencing reiterated within primary flower buds appearing in inflorescences emerging from secondary shoots.

### EXAMPLES

### EXAMPLE 1

### Stable transformation of tobacco plants

Transgenic *Nicotiana tabacum* var. SR1 plants were obtained by leaf disc transformation with the *Agrobacterium tumefaciens* strain LB4004, harbouring a binary vector, with the method described by Horsch RB et al, 1985, Science 227: 1229-1231.

### EXAMPLE 2

### Suppression of chromosome recombination in vivo through long-distance transport of siRNA across a graft junction in tobacco

An important example of a process that typically occurs in the male meiocytes of a plant is meiotic chromosome recombination. This invention allows the interference with meiotic chromosome recombination by means of a transgene in a rootstock that generates short interfering RNA (siRNA) molecules, which are delivered into the male meiocytes that are produced by a scion grafted on top of the transgenic rootstock.

To provide evidence for this, transgenic tobacco plants (*Nicotiana tabacum* var. SR1) harbouring an ectopically expressed RNAi hairpin construct for the *DMC1* gene (*DISRUPTED MEIOTIC cDNA1*) from *Arabidopsis thaliana* (TAIR: AT3G22880; expression driven by a constitutive promoter) were produced following the method of example 1. *DMC1* is a meiotic cell-cycle-specific factor and a member of the highly conserved RecA-type recombinase family (DNA dependent ATP-ases) present in meiocytes, such as pollen mother cells in anthers and megaspore mother cells in ovules (Doutriaux MP et al, 1998, Mol Gen Genet 257: 283-291). *DMC1* was shown to be crucial for inter-homolog recombination and bivalent formation, and, consequently, for proper pollen and ovule development. Lack of a functional *DMC1* gene product induces achiasmatic meiosis, resulting in formation of anomalous pollen grains, which contain an aberrant number of chromosomes. The chromosome number in spores depends on an orderly meiotic separation of sister chromatids occurring in the second meiotic phase.

The plants were grown in soil in greenhouse conditions (16 hours of light, 25°C day temperature and 20°C night temperature), and three transgenic lines clearly showing the silencing phenotype of *DMC1* were selected for further experiments. This silencing phenotype comprises consistently low levels of endogenous *DMC1* mRNA, the formation of unbalanced microspore tetrads after meiosis, at least partial male sterility and the production of irregularly shaped (aneuploid) pollen, which are indicative for a dysfunctional meiosis, caused by the presence of siRNA molecules targeting the *DMC1* transcript in the male meiocytes.

Scions from wildtype plants were grafted onto 3-to 4-week-old plants from these selected transgenic lines (with the first leaf being about 10 cm in length). Two plants are thus combined, in which the bottom part (transgenic rootstock) of the one plant supports the upper part (wildtype scion) of the other plant. The graft incisions were made at an angle of 45° with a razor blade, usually between the third and fourth leaf. During the transfer the scion stem was shortly submerged in water, until the graft junction was aligned. A toothpick served as space holder between the parafilm and wrapped stem to assist some evaporation from the graft junction.

After grafting, newly emerging leaves on the scion and secondary shoots appearing on the rootstock were cut off, to force the delivery of phloem-mediated molecules towards the apical region of the scion. A successful graft namely leads to the formation of a vascular connection between the rootstock and the scion, and phloem transport is directed towards sink tissues (such as meristematic regions and newly emerging leaves). By ensuring that the apical region of the scion is the only remaining above-ground sink tissue in the plant, phloem transport can be effectively directed towards that apical region, where flowers will be formed. Control grafts were included in the experiment, namely wildtype scions grafted onto wildtype rootstocks, and transgenic scions grafted onto transgenic or wildtype rootstocks.

Bolting of the scions occurred approximately 3 weeks after grafting. Preliminary examination of the anthers revealed a deficiency in pollen production in all grafts in which either the scion or the rootstock were transgenic **(****Figure 1B****).** This phenotype was consistent with the silencing phenotype of *DMC1.* In the former case this was not surprising, as the entire scion was transgenic, and inside the meiocytes siRNA molecules directed against *DMC1* were thus produced. In the latter case, however, it implied that - as taught by the current invention - siRNA molecules generated by the RNAi hairpin construct directed against *DMC1* were mobilized from the transgenic rootstock and transported systemically into the scion with the phloem sap, and delivered into the male meiocytes that were produced inside sporogenic tissues of the flower buds of the scion.

In control grafts in which the scion itself was transgenic this male sterility was observed in all flowers, while in grafts in which the scion was wildtype (grafted onto a transgenic rootstock) the effect was limited to the first flowers. First flowers were nearly sterile, while later flowers were fully fertile. The phloem-mediated siRNA signal thus preferentially entered the initially produced flowers, being the first sink tissue the phloem sap encountered on its way towards the scion's apex. The male sterility phenotype was also observed in the first flowers that developed on secondary shoots.

A more thorough examination of the first flowers from wildtype scions grafted onto a transgenic rootstock was conducted, and - based on a highly reproducible relation between size distribution and fluorescence emission from wildtype pollen - fluorescence-activated cell sorting (FACS) analysis revealed a much higher variability in pollen size (irregular distribution), as compared to pollen from wildtype flowers **(****Figure 1B****).**

This observation was confirmed with scanning electron microscopy, and pollen produced by the first flowers of wildtype scions supported by a *DMC1* siRNA-producing transgenic rootstock appeared misshapen and smaller. Pollen from later flowers was normal **(****Figure 2****).**

**Table 1 Statistics of DMC1 siRNA phenotypes observed after grafting.**

| **Stock / scion** | ***DMC1* phenotype/Grafts tested^{a}** |
|---|---|
| wild type/wild type | 0/13 (0%) |
| wild type/*DMC1* siRNA | 15/15 (100%) |
| *DMC1* siRNA/wild type | 6/13 (46%) |
| wild type/*YFP*-LN *DMC1* | 0/8 (0%) |
| *YFP-ΔN DMC1*/*DMC1* siRNA | 5/5 (100%) |
| *DMC1* siRNA/*YFP*-ΔN *DMC1* | 3/5 (60%) ^{b} |

| | |
|---|---|
| **^{a}** evaluated by sterility, aberrant pollen production, appearance of unbalanced tetrads, FACS, cytological inspection of meiosis, qPCR, and presence of *DMC1-*specific siRNA molecules. **^{b}** verified by lack of *YFP*-ΔN *DMC1* fluorescence In scion leaves and primary buds. | |

To further address this phenotype, an earlier stage in pollen development was investigated. In control grafts (wildtype scion onto wildtype rootstock) microspore tetrads were all normal-looking and balanced (n=100). In wildtype scions grafted onto transgenic rootstocks, however, the overall number of tetrads was lower in first (early) flowers, and a significant number of these tetrads (up to 10%, with n>100) was unbalanced, as indicated by the appearance of a small nucleus in one tetradic cell (figure 2). Unbalanced tetrad formation is one of the typical phenotypes associated with the loss of *DMC1* activity during meiosis.

While *DMC1* is also active in the female reproductive organs, no evidence of *DMC1* silencing was apparent in the female reproductive organs of wildtype scions grafted onto transgenic rootstocks. When pollinated with wildtype pollen they produced a normal number of viable seeds, indicating a normal female fertility.

To further investigate the *DMC1* silencing in male meiocytes through systemic transport of RNAi signals, another set of transgenic tobacco plants was generated, using the method described in Example 1. These plants harboured a *35S*::*YFP:ΔN-DMC1* transgenic reporter construct, containing a dysfunctional, truncated *DMC1* cDNA of *Arabidopsis thaliana* lacking the first 276 bp (i.e. leading to deletion of 92 amino acids at the N-terminus), fused to the Yellow Fluorescent Protein (YFP) which serves as a visual reporter, and driven by the constitutive cauliflower mosaic 35S promoter (CaMV 35S).

Shoots from these transgenic plants - which normally show a ubiquitous fluorescent signal due to the overexpression of the fusion protein - were subsequently grafted onto transgenic rootstocks harbouring the *DMC1* silencing construct. The flower buds emerging on the scion were examined, and in the first (early) flower buds the YFP fluorescence signal was strong in carpels and stigma, but absent inside anthers. This demonstrated that in primary flower buds the systemic silencing signal is preferably delivered into anthers **(****Figure 3****).**

Thus, this example teaches how chromosome recombination can be efficiently suppressed in male meiocytes of tobacco, without rendering these meiocytes or the male gametes resulting thereof transgenic. This invention allows the application of Reverse Breeding (WO03017753) in a preferred embodiment, namely with non-transgenic progeny. This concept is further explained in Example 3.

While in this example the *DMC1* gene was targeted, any other gene that is functionally involved in any of the steps of meiotic chromosome recombination could be targeted with comparable results.

### EXAMPLE 3

### Non-transgenic Reverse Breeding in Brassica, mediated by systemic silencing signals

The experimental approach outlined in Example 2 was applied to *Brassica oleracea,* resulting in transgenic rootstocks harbouring an ectopically expressed hairpin RNAi silencing construct against the *SDS* (*SOLO DANCERS*) gene. Onto these transgenic rootstocks wildtype hybrid scions were grafted from an interspecific F1 hybrid (*Raphanus sativus* x *Brassica oleracea*), and the silencing signal was forced into male meiocytes by removing all scion leaves, as described in Example 2. The first flower buds produced by the scions contained a significant proportion of aberrant microspore tetrads, and these flowers were largely male sterile.

When the first flower buds produced on the scion were about 3 mm in size, the microspores developing therein had reached the optimal stage for microspore regeneration in vitro (androgenesis). Microspores were purified from these buds, and they were given a stress treatment of two days at 32°C, which is optimal to induce sporophytic development of the haploid spores. Balanced (euploid) microspores were regenerated into haploid plantlets using methods known to the person skilled in the art (for example Swanson EB et al., Plant Cell Rep 6: 94-97; Takahata Y and Keller WA, Plant Sci 74: 235-242), and colchicine treatment was subsequently used for genome doubling, leading to haploid or doubled haploid (DH) plantlets with a full complement of chromosomes.

Due to the suppression of chromosome recombination by *DMC1* silencing in the male meiocytes, the chromosomes in the DH plants obtained by this method had not been recombined, and they had the exact genetic constitution as the chromosomes of the parental lines of the hybrid plant that had been used as a scion for grafting. This was confirmed with molecular markers (two per chromosome, i.e. one per chromosome arm) that are polymorphic between *Raphanus sativus* and *Brassica oleracea,* the parents of the F1 hybrid that was used as a scion in the graft.

Using the same set of molecular markers, DH plants regenerated from microspores collected from anthers of the scion were screened to identify individuals that were genetically complementary, i.e. containing sets of parental chromosomes that when combined in an F1 correspond to the exact set of chromosomes present in the hybrid used as a scion for grafting. Crossing of these complementary individuals resulted in the exact reconstruction of the hybrid genotype (*Raphanus sativus* x *Brassica oleracea*).

This example thus illustrates how the current invention can be used to exactly reconstruct a hybrid genotype. Importantly, this was achieved without rendering the reconstructed hybrid plant transgenic, as suppression of chromosome recombination prior to DH production had been achieved through a transient siRNA signal in the male meiocytes.

The approach outlined in the previous examples can also be applied in other plant species. It is also particularly useful to apply it in crop species with long life cycles, such as trees (for example poplar, and fruit trees such as apple, pear, cherry, peach, plum, banana, citrus fruits, papaya, fig, etc.) and grape vines. This invention could be used to seed-propagate specific traits in such crops, and transgene-free elite lines can be produced much faster, and several years of traditional breeding efforts can be avoided.

## Claims

1. Method for interfering with processes in the male meiocytes of a plant, by combining a first plant comprising a rootstock, which transgenically harbours a nucleic acid sequence, with a scion from a second plant grafted onto the rootstock of the said first plant, whereby the said transgenic nucleic acid sequence generates polynucleotide molecules in the rootstock of the first plant, which polynucleotide molecules are transported systemically across the graft junction to enter the male meiocytes produced by the scion of the second plant, and wherein the said systemically transported polynucleotide molecules are at least partially complementary to a transcript expressed in the male meiocytes produced by the scion of the second plant.

2. Method as claimed in claim 1, wherein expression of the transgenic nucleic acid sequence in the first plant is achieved by operably linking the said nucleic acid sequence to a promoter sequence that confers a ubiquitous expression profile, or a tissue- or cell-type-specific expression profile, onto the said transgenic nucleic acid sequence.

3. Method as claimed in claim 2, wherein the promoter sequence confers onto the said transgenic nucleic acid sequence an expression profile that encompasses roots.

4. Method as claimed in claims 1-3, wherein interfering with processes in the male meiocytes consists of the suppression or blocking of processes in the male meiocytes.

5. Method as claimed in claim 4, wherein the suppression or blocking of processes in the male meiocytes is achieved by destabilizing the target gene mRNA by means of polynucleotide molecules that are at least partially complementary to the target gene mRNA or transcript and that have been generated from the transgenic nucleic acid sequence.

6. Method as claimed in claim 5, wherein the polynucleotide molecules are selected from the group comprising miRNA, antisense RNA, RNAi molecules, siRNA molecules, Virus-Induced Gene Silencing (VIGS) molecules, co-suppressor molecules or RNA oligonucleotides.

7. Method as claimed in claims 1-3, wherein influencing of processes in the male meiocytes of plants consists of the enhancement of processes in the male meiocytes of plants.

8. Method as claimed in claims 1-7, wherein the polynucleotide molecules are transiently expressed in the first plant.

9. Method as claimed in claim 8, wherein the transient expression of the polynucleotide molecules in the first plant is achieved by use of an inducible promoter, selected from the group comprising heat-inducible promoters, chemical-inducible promoters, steroid-inducible promoters and alcohol-inducible promoters.

10. Method as claimed in claim 8, wherein the transient expression of the polynucleotide molecules is effected by expression from a construct encoding the polynucleotide molecules that is not integrated in the genome of the rootstock cells.

11. Method as claimed in claim 10, wherein construct is delivered to the rootstock cells by infiltration of leaves on the rootstock with *Agrobacterium.*

12. Method as claimed in claims 1-11, wherein the polynucleotide molecules are at least partially complementary to the transcript of a gene that is involved in chromosome recombination.

13. Method as claimed in claim 12, wherein the gene is selected from the group comprising *SPO11, MER1, MER2, MRE2, MEI4, REC102, REC104, REC114, MEK1*/*MRE4, RED1, HOP1, RAD50, MRE11, XRS2, PRD1, PRD2, PRD3, PHS1, NBS1, COM1, RHD54*/*TID1, DMC1, SAE3, RED1, HOP1, HOP2, MND1, REC8, MEI5, RAD51, RAD52, RAD54, RAD55, RAD57, RPA1, SMC3, SCC1, MSH2, MSH3, MSH6, PMS1, SOLODANCERS, HIM6, CHK2, SGS1, MSH4, MSH5, MER3*/*RCK, ZIP1, ZIP2, ZIP3, ZIP4, PTD, SHOC1, ZYP1, MLH1, MLH3,* or their functional homologues.

14. Method as claimed in any one of the claims 1-13, wherein the polynucleotide is a DNA polynucleotide.

15. Method for specifically altering the genome sequence of male meiocytes of a plant, by combining a first plant comprising a rootstock, which transgenically harbours a nucleic acid sequence, with a scion from a second plant grafted onto the rootstock of the said first plant, whereby the said transgenic nucleic acid sequence generates polynucleotide molecules in the rootstock of the first plant, which polynucleotide molecules are transported systemically across the graft junction to enter the male meiocytes produced by the scion of the second plant, and wherein the said polynucleotide molecules are at least partially homologous to a DNA fragment contained in the genome of the second plant.
